# FASCICULE DE BREVET EUROPEEN

(11) **EP 1 680 190 B1**
(45) Date de publication et mention de la délivrance du brevet: **02.12.2009**
(21) Numéro de dépôt: 04791473.4
(22) Date de dépôt: 06.10.2004
(51) Int. Cl.: A61Q 13/00, A61Q 17/02, A61Q 19/00, C11B 9/00, A61K 8/34, A61K 8/36, A61K 8/39, A61K 8/92, A61K 8/97

(54) **Procédé de traitement non thérapeutique POUR ANIMAUX DE COMPAGNIE.**
Nicht-therapeutische Behandlungsmethode FÜR HAUSTIERE
Non-therapeutic treatment process FOR PETS

(30) Priorité: 06.10.2003 FR 0311687
(43) Date de publication de la demande: 19.07.2006
(60) Demande divisionnaire: 07121305.2
(73) Titulaire: Laboratoire de Dermo-Cosmetique Animale, 81100 Castres (FR)
(72) Inventeur: FABRIES, Lionel, F-81100 Castres (FR)
(74) Mandataire: Sartorius, Jérome
(86) Numéro de dépôt international: PCT/FR2004/002518
(87) Numéro de publication internationale: WO 2005/034898

(56) Documents cités:
- EP-A- 0 173 478
- EP-A- 0 278 809
- EP-A- 0 607 886
- EP-A- 0 900 561
- WO-A-02/067878
- FR-A- 2 681 783
- US-A- 5 871 757
- DATABASE WPI Derwent Publications Ltd., London, GB; AN 1996-220169 XP002280942 VOLFENZONI I ET AL.: "Face cream" & RU 2 043 764 C (SYNTH.NAT.FRAGRANCES RES.INST.) 20 septembre 1995 (1995-09-20)
- PEPE R C, ET AL (EDS.): "International Cosmetic Ingredient Dictionary and Handbook, Vol.1" 2002, COSMETIC, TOILETRY AND FRAGRANCE ASSOCIATION , WASHINGTON DC, USA , XP002280940 pages 600-601
- NN: "Definitionen und Literatur" EAU DE COLOGNE, [Online] page 1, XP002280937 Extrait de l'Internet: URL:http://www.eau-de-cologne.com/literatu r.html>
- NN: "Canna Body Deo" HANF INFO, [Online] page 1, XP002280938 Extrait de l'Internet: URL:http://www.chanvre-info.ch/shop/index. php?pro=043004&cat=4>
- NATURPRÄPARATE G. DIETERICH GMBH: "Franzbranntwein - Gel - Mückenbalsam" HOLZHACKER, [Online] pages 1-3, XP002280939 Extrait de l'Internet: URL:http://www.holzhacker.de/produkte/sort iment/Franzbranntwein/620191-detail.html> & déclaration INCI "Holzhacker Franzbranntwein Gel" sur carton 2001
- DATABASE WPI Derwent Publications Ltd., London, GB; AN 2003-477505 XP002280943 CHAE H N, ET AL: "Cosmetic composition containing extract of grapefruit seed, radix sophorae flavescentis and azadirachta indica and having enhanced antimicrobial effect" & KR 2003 017 295 A (NOWCOS CO.) 3 mars 2003 (2003-03-03)
- FALBE J, REGITZ M (EDS.): "Römpp Chemie Lexikon" 1995, THIEME , STUTTGART , XP002280941 page 961 page 1035

## Description

La présente invention concerne un procédé de traitement non thérapeutique pour animaux de compagnie (y compris les nouveaux animaux de compagnie NAC).

Les produits de dermatologie vétérinaire à but préventif ou d'entretien de l'animal sont conçus généralement sous forme de crèmes, ou de lotions ou de shampooings.

Leur application à l'animal est toujours délicate et parfois désagréable pour celui-ci.

La tendance normale de l'utilisateur est souvent de surdoser la quantité de produit appliqué, c'est le cas fréquent des lotions ou des poudres, ce qui a pour effet de perturber l'animal.

Les poudres, shampooings ou lotions sont souvent à effet localisé, ce qui ne permet pas de couvrir par une seule application l'ensemble des besoins d'un traitement Dermo-Cosmétique :
- Soin de la peau et hydratation de celle-ci,
- Effet désodorisant,
- Effet assainissant et purifiant,
- Effet antioxydant et anti-radicalaire,

La présente invention vise à obvier à ces inconvenients au moyen d'un procédé selon les revendications 1 à 13.

La composition appliquée permet après une application localisée la diffusion des principes actifs sur toute la surface grâce au solvant vecteur de diffusion puis l'invention met en oeuvre la capacité des glandes sébacées à stocker les principes actifs du traitement et à les relarguer progressivement par la production naturelle de sébum.

A cet effet, la composition Dermo-Cosmétique mettant en oeuvre la capacité des glandes sébacées à stocker le ou les principe(s) actifs et à les relarguer et à les diffuser par le sébum se caractérise essentiellement en ce qu'elle comprend au moins :
❖ Un vecteur qui est un solvant.
❖ Un complexe d'huiles essentielles choisies seules ou en combinaison entre elles en fonction des effets à obtenir.

Cette combinaison permet de proposer une composition prête à l'emploi, essentiellement d'un vecteur qui est un solvant et d'huiles essentielles qui, appliquée en un ou plusieurs points du pelage de l'animal, réalise un traitement sur l'intégralité de la surface cutanée de l'animal pendant plusieurs jours. Des essais réalisés ont démontré l'obtention d'un résultat sur 7 jours au moins.

Suivant une autre caractéristique de l'invention, le solvant constituant Vecteur est un ethoxydiglycol.

Suivant une autre caractéristique, la composition comprend :
❖ Un vecteur constitué d'un solvant organique,
❖ Un complexe d'huiles essentielles,
❖ Des acides gras poly-insaturés, utilisés par voie locale chez les animaux.

D'autres avantages et caractéristiques de l'invention apparaîtront à la lecture de la description ci après.

La composition selon l'invention vise à réaliser un traitement de la peau de l'animal qui ait plusieurs effets :
- Hydratation ;
- Effet désodorisant ;
- Effet assainissant et purifiant ;
- Effet antioxydant et anti-radicalaire ;

L'intérêt de l'invention est de pouvoir conjuguer tout ou partie des effets ci-dessus, selon la complexité de la composition à partir d'une application sur une zone réduite de l'épiderme de l'animal.

L'invention est basée sur l'association d'une synergie aromatique unique et novatrice à d'autres actifs hydratants, antioxydants et nourrissants de la peau.

Chacune des huiles essentielles entrant dans la composition fait l'objet d'une définition qualitative précise basée sur les critères EOBBD (Essential Oil Botanically and Biochemically defined, iso 9002 certified Quality Assurence Process).

Une seule application suffit pour un animal de petite taille ; deux seront nécessaires pour un animal de plus grande taille.

Suivant une formulation basique, la composition comprend au moins un vecteur qui est constitué d'un solvant organique ou d'un équivalent et un complexe concentré d'au moins deux huiles essentielles.

La présence du vecteur permet l'application de la composition en un ou deux points de la surface cutanée, la diffusion du produit, son stockage par les glandes sébacées et son relargage progressif.

A ce jour, la capacité du vecteur n'était utilisée que pour apporter à l'animal un produit insecticide.

L'intérêt de l'invention est de proposer un traitement complet de l'épiderme et du pelage qui ait un effet assainissant, et calmant sur l'animal :
- Par la restauration d'un écosystème cutané équilibré.
- La composition apporte à l'animal une sensation de bien être et de fraîcheur qui est régulatrice de son humeur.

L'originalité de la composition permet de baigner l'animal 48 heures avant ou après le traitement, tout en bénéficiant de l'efficacité de la composition appliquée pendant une semaine au moins.

Le solvant organique constituant le vecteur peut être de préférence un solvant de polarité intermédiaire par exemple :
➢ Un ethoxydiglycol ou un butyleneglycol, ou un propyléneglycol, ou un glycérine dans la proportion des 1 % à 99 % de la composition totale en fonction de ses autres constituants.

Plus généralement, le vecteur est choisi parmi le groupe :
Acétone, acétonitrile, alcool benzylique, butyldiglycol, diméthylacétamide, diméthyformamide, éther n-butylique du dipropylèneglycol, éthanol, isopropanol, méthanol, éthylèneglycol monoéthyléther, monométhylacétamide, monométhyléther de dipropylèneglycol, polyoxyéthylène-glycols liquide, propyléneglycol, 2-pyrrolidone, notamment N-méthyl pyrrolidone, monoéthylèther de diéthylèneglycol, éthylèneglycol, diéthylphtalate, et mélange d'au moins deux d'entre eux.

La composition ci-dessus peut-être complétée par des acides gras essentiels poly-insaturés pour l'amélioration de la surface cutanée.

Les acides gras poly-insaturés oméga 3 et oméga 6 peuvent être apportés par l'adjonction d'huile de graine de chanvre (cannabis sativa) dans une proportion qui peut être de 1% à 15% de la composition totale ou par des substituts naturels tels que :
- huile de poisson (orange Roughy),
- huile de Bourrache (Borago Officinalis),
- huile de coco (Cocos Nucifera),
- huile de Kukui,
- huile de sésame (Sesamum Indicum),
- AGPI de synthèse reconstitués ou extraits naturels,
- Acide Eicopentaenoïque (EPA) et
- Acide Docosahexaenoïque (DHA),
- Beurre de karité (Shea Butter),
- Huile d'olive (Olea Europea),
- Huile de colza,
- Huile de noix,
- Huile de Soja,
- Huile de Buglosse (echium Plantagineum),

Qui peuvent être ajoutés à la composition dans la proportion de 1 à 99 %, plus particulièrement de 2 % à 50 % et de préférence de 5 % à 10 % de la composition totale.

La composition comprend également en combinaison avec l'un quelconque des composés ci-dessus un agent filmogène inhibiteur de cristallisation épaississant et stabilisant tel que le produit polivinylpyrrolidone ou choisi parmi le groupe.

Hydroxyméthyl cellulose, alcools polyvinyliques, copolymères d'acétate de vinyle et de vinylpyrrolidone, polyéthyléneglycols, alcool benzylique, mannitol, glycérol, sorbitol, esters de sorbitane polyoxyéthylénés, lécithine, carboxyméthylcellulose sodique, dérivés acryliques tels que méthacrylates dérivés des polyesters du type des copolymères triméthylpentanedjol / acide adipique et autres.

Plus généralement, la composition peut comporter seule ou en combinaison les huiles essentielles suivantes :
o Rosmarinus officinalis dans la proportion de 0,3% à 0,9% qui a un effet assainissant purifiant et apaisant ou de l'Eucalyptol.
o Lavandula hybrida dans la proportion de 0,3% à 0,9% ou lavandula angustifolia qui a un effet désodorisant.
o Eugenia caryophyllus qui a un effet répulsif pour les insectes, dans la proportion de 0,3% à 0,9%.
o Cinnamorum Camphora ou Camphor, dans la proportion 0,15% à 0,6% à effet désodorisant et antiprurigineux.
o Mentha Piperita ou mentha orvensis dans la proportion de 0,15% à 0,6% à effet désodorisant.
o Gaultheria procumbens ou son substitut méthylsalicylate à effet calmant et antiprurigineux.
o Musk synthétique ou musk ketone, dans la proportion de 0,06% à 0,3% pour parfumer ou masquer les odeurs naturelles.

Les composés ci-dessus sont utilisés en combinaison dans la composition en fonction des effets recherchés.

Plus généralement, les huiles essentielles de la liste jointe peuvent être combinées à la composition

| **SUBSTITUTS COMPLEXE D'HUILES ESSENTIELLES** | | | | |
|---|---|---|---|---|
| Ajowan | Amandier amer | Ammi, khella | Aneth odorant | Angélique |
| Armose herbe blanche | Basilic | Bay | Bergamotier | Bois de rose |
| Bois de santal | Boldo | Bucchu | Cabreuva | Cajeput |
| Calamus | Camomille romaine ou Noble | Camphrier | Cannelier de Ceylan | Cannelle de Chine |
| Cardamones | Carotte | Carvi | Cataire | Cèdre de l'Atlas |
| Cèdre de Virginie | Céleri | Chénopode vermifuge | Ciste ladanifére | Citron jaune |
| Citronnelle de Ceylan | Citronnelle de Java | Copahu | Coriandre | Cubèbe |
| Cumin | Curcuma | toujours vert | Cyprès Douglas | Elémi |
| Encens | Epinette noire | Estragon | Eucalyptus | Gaiac |
| Galbanum | Gaulthérie, Wintergreen | Genévrier commun | Géranium odorant | Gingembre |
| Giroflier | Hélichryse | Hysope à rameaux couchés | Hysope officinale | Inule |
| | | | | |
| Iorménie, Camomille sauvage | | Lantane | Laurier noble | Lavande aspic |
| Lavande officinale | | Lavande stoechade | Lavandin | Lemon-grass |
| Lentisque pistachier | | Lepstosperme citronné | Limette | Litsée |
| Livèche | | Mandarine rouge | Marjolaine à coquilles | Marjolaine sylvestre |
| Marjolaine vivace | | Matricaire | Mélèze | Mélisse véritable |
| Menthe des champs | | Menthe poivrée | Menthe pouliot | Millefeuille |
| Millepertuis | | Muscade | Myrrhe | Myrte |
| Nard | | Niaouli | Oranger | Oranger doux |
| Origan | | Origan d'Espagne | Origan vulgaire | Palma - Rosa |
| Pamplemousse | | Patchouli | Persil | Pin des Landes |
| Pin Sylvestre | | Poivre noir | Vinylpyrrolidone aromatique | Romarin officinal |
| Romarin pyramidal | | Rose de Bulgarie | Santal amyris | Santoline petit cyprès |
| Sapin baumier | | Sapin de Sibérie | Sapin pectiné | Sarriette vivace |
| Sassafras | | Sauge à petite feuille | Sauge officinale | Sauge sclarée |
| Serpolet | | Tanaisie bleue | Tea tree | Térébenthine |
| Thuya | | Thym | Thym à feuilles de sarriette | Verge d'or |
| Vergerette | | Verveine odorante | Vétiver | Ylang-ylang, distillation complète |
| Ylang-ylang, tête de cuvée | | | | |

Les huiles essentielles sélectionnées l'ont été en fonction d'un cahier des charges précis quant aux principes actifs qu'elles contiennent.

Les principes actifs de la formulation pourront être intégralement ou partiellement inclus dans une formulation « retard » permettant un allongement du temps de relargage et donc de la durée d'action de ces principes actifs. Les composés permettant cette modification pourront être choisis parmi les bêta-cyclodextrines, la silice, les fibres de cellulose, le nylon, les poly-méthacrylates.

Une formule retard ou à libération programmée peut permettre une libération prolongée des principes actifs jusqu'à vingt-huit jours après l'application. Les formules retard font usage de :
- Bêta cyclodextrines, sucres cycliques d'origine naturelle : présents dans la formulation en concentration variant de 2 à 10 %.
- Et/ou fibres de cellulose, originaires du bois : présents dans la formulation en concentration variant de 2 à 10 %.
- Et/ou sphères de polyméthyleméthacrylate et de nylon : présents dans la formulation en concentration variant de 4 à 12 %.
- Et/ou microréservoirs de silice, enrobés ou non enrobés : présents dans la formulation en concentration variant de 2 à 10 %.
- Et/ou polymères polyesters linéaires de masse moléculaire de 800 à 5000 occlusifs Daltons et retensifs : présents dans la formulation en concentration variant de 10 à 20 %.

Les proportions de chacun des composants sont prises dans les valeurs de pourcentages énoncés plus haut, ces valeurs étant exprimées en pourcentage de la composition totale et complète.

## Revendications

1. Procédé de traitement non thérapeutique comportent les étapes suivantes:
a) application en un ou deux points de la surface cutanée d'un animal à pelage d'une composition dermo-cosmétique mettant en oeuvre la capacité des glandes sébacées à stocker des principes actifs et à les relarguer par le sébum, ladite composition comprenant au moins:
- un solvant vecteur de difference des principes actifs de ladite composition à ladite surface cutanée, et
- au moins un complexe d'huiles essentielles,
b) diffusion des principes actifs de ladite composition,
c) stockage des principes actifs de ladite composition par les glandes sébacées et relargage progressif.

2. Procédé selon la revendication 1 **caractérisé en ce que** le solvant vecteur est un éthoxydiglycol.

3. Procédé selon la revendication 1, **caractérisé en ce que** le solvant vecteur est choisi parmi le groupe:
Acétone, acétonitrile, alcool benzylique, butyldiglycol, diméthylacétamide, diméthyformamide, éther n-butylique du dipropylèneglycol, éthanol, isopropanol, méthanol, éthylèneglycol monoéthyléther,monométhylacétamide, monométhyléther de dipropylèneglycol, polyoxyéthylène-glycols liquide, propyléneglycol, 2-pyrrolidine, monoéthylèther de diéthyléneglycol, éthyléneglycol, diéthylphtalate, et mélange d'au moins deux d'entre eux.

4. Procédé selon les revendications 1, 2, et 3, caratérisé en ce que la composition comprend:
un vecteur constitué d'un solvant
organique,
un complexe d'huiles essentielles,
des acides gras poly-insaturés, utilisés par voie
locale chez les animaux.

5. Procédé selon la revendication 4, **caractérisé en ce que** les acides gras sont choisis dans les huiles de chanvre, dans la proportion de 1 à 15%.

6. Procédé selon la revendication 4, **caractérisé en ce que** les acides gras sont apportés par des produits choisis dans le groupe :
Huile de poisson (orange Roughy)
Huile de Bourrache (Borago Officinalls)
Huile de coco (Cocos Nucifera)
Huile de Kukui
Huile de sésame (Sesamum Indicum)
AGPl de synthèse reconstitués ou extraits naturels.
Acide Elcopentaenoïque (EPA) et
Acide Docosahexaenoïque (DHA),
Beurre de karité (Shea Butter)
Huile d'olive (Olea Europea)
Huile de colza
Huile de noix
Huile de Soja
Huile de Buglosse (echlum Plantagineum)
dans la proportion de 3 à 50 %

7. Procédé selon la revendication 6, dans lequel la proportion desdits acides gras est compris entre 5 et 10 %.

8. Procédé selon l'une quelconque des revendications précédentes, **caractérisé en ce que** la composition comprend un agent filmogène inhibiteur de cristallisation, épaississant et stabilisant.

9. Procédé selon la revendication précédente, dans lequel l'agent filmogéne est une Polivinylpyrrolidone ou est choisi parmi le groupe :
hydroxymethyl cellulose, alcools polyvinyliques, copolymères d'acétate de vinyle et de vinylpyrrolidoné, polyéthylénéglycols, alcool benzylique, mannitol, glycérol, sorbitol, esters de sorbitane polyoxyéthylénés, lécithine, carboxyméthylcellulose sodique, dérivés acryliques tels que méthacrylates et autres.

10. Procédé selon l'une quelconque des revendications précédentes, **caractérisé en ce que** la composition comprend une formule retard permettant une libération prolongée des principes actifs.

11. Procédé selon la revendication précédente, **caractérisé en ce que** ladite formule retard fait usage de:
Silice et/ou
Bêta cyclodextrines, sucres cycliques d'origine naturelle présents dans la formulation en concentration variant de 2 à 10%
Et/ou fibres de cellulose, originaires du bois : présents dans la formulation en concentration variant de 2 à 10 %,
Et/ou sphères de polyméthyleméthacrylate et de nylon: présents dans la formulation en concentration variant de 4 à 12 %,
Et/ou microréservoirs de silice, enrobés ou non enrobés: présents dans la formulation en concentration variant de 2 à 10 %,
Et/ou polymères polyesters linéaires de masse moléculaire de 800 à 5000 Daltons occlusifs et rétensifs : présents dans la formulation en concentration variant de 10 à 20 %.

12. Procédé selon l'une quelconque des revendications précédentes, déterminé de maniére que, quand la composition dermo-cosmétique est appliquée en un ou plusieurs points du pelage de l'animal, elle réalise un traitement sur l'intégralité de la surface cutanée de l'animal pendant plusieurs jours.

13. Procédé selon l'une quelconque des revendications précédentes dans lequel l'animal est un animal de compagnie.

## Claims

1. Non therapeutic treatment method comprising the following steps:
a) application, in one or more locations of the skin surface of a furry animal, of a dermo-cosmetic composition using the capacity of the sebaceous glands to store the active ingredients and to release them through the sebum, said composition comprising at least:
- a carrier solvent to diffuse active ingredients of said composition at said skin surface, and
- at least one complex of essential oils,
b) diffusion of the active ingredients of said composition,
c) storing of the active ingredients of said composition by the sebaceous glands and gradual releasing.

2. Method according to claim 1 **characterized in that** the solvent making up the carrier is an ethoxydiglycol.

3. Method according to claim 1, **characterized in that** the carrier solvent is chosen from the following group: Acetone, acetonitrile, benzyl alcohol, butyldiglycol, dimethyl acetamide, dimethyl formamide, dipropylene glycol N-butyl ether, ethanol, isopropanol, methanol, ethylene glycol monoethyl ether, monomethyl acetamide, dipropylene glycol monomethyl ether, liquid polyoxyethylene-glycol, propylene glycol, 2-pyrrolidone, diethyelene glycol monoethyl ether, ethylene glycol, diethyl phtaiate, and a mixture of at least two of them.

4. Method according to claims 1, 2 and 3 **characterized in that** the composition comprise:
- A carrier consisting of an organic solvent,
- A complex of essential oils,
- Polyunsaturated fatty acids used locally in animals.

5. Method according to claim 4, **characterized in that** the fatty acids are chosen from hemp oils, in the proportion of 1 to 15%.

6. Method according to claim 4 **characterized in that** the fatty acids are added using products chosen from the following group:
- fish oil (orange roughy),
- borage oil (Borago Officinalis),
- coconut oil (Cocos Nucifera),
- kukui oil,
- sesame oil (Sesamum indium),
- reconstituted synthetic AGPl's or natural extracts.
- Eicopentaenoic acid (EPA) and
- docosahexaenoic acid (DHA),
- shea butter,
- olive oil (Olea Europea),
- canola oil,
- walnut oil,
- soybean oil,
- anchusa oil (Echium Plantagineum),
in the proportion of 3 to 50%.

7. Method according to claim 6 **characterized in that** the proportion of said fatty acids ranges between 5 and 10%.

8. Method according to any of the preceding claims, **characterized in that** the composition contains a thickening and stabilizing crystallization-inhibiting film-forming agent.

9. Method according to the preceding claim in which the film-forming agent is a polyvinyl pyrrolidone or a product chosen from the following group: hydroxymethyl cellulose, polyvinyl alcohols, vinyl acetate and vinyl pyrrolidone copolymers, polyethylene glycols, benzyl alcohol, mannitol, glycerol, sorbitol, polyoxyethylene sorbitan esters, lecithin, sodium carboxymethyl cellulose, acrylic derivatives such as methacrylates and others.

10. Method according to any of the preceding claims, **characterized in that** the composition contains a delay formula allowing for extended release of the active ingredients.

11. Method according to the preceding claim, **characterized in that** the delay formulas use:
- Silica and/or
- Beta-cyclodextrins, cyclic natural sugars: presents in the formulation with a concentration ranging from 2 to 10%,
- And/or cellulose fibers from wood: present in the formulation with a concentration ranging from 2 to 10%,
- And/or polymethyl methacrylate and nylon spheres: present in the formulation with a concentration ranging from 4 à 12%,
- And/or coated or non-coated silica microreservoirs: present in the formulation with a concentration ranging from 2 to 10%,
- And/or linear polyester polymers with a molecular weight of 800 through 5000 Daltons both occlusive and retentive: present in the formulation with a concentration ranging from 10 to 20%.

12. Method according to any of the preceding claims, determined so that, when the dermo-cosmectic composition is applied in one or more locations of the skin surface of the fur of the animal, it achieves treatment over the whole skin surface of the animal for several days.

13. Method according to any of the preceding claims in which the animal is a pet.

## Patentansprüche

1. Verfahren zur nichttherapeutischen Behandlung mit den folgenden Stufen:
a) Auftragen auf einer oder zwei Stellen der Hautoberfläche eines Tieres mit Fell einer hautkosmetischen Zusammensetzung, welche die Fähigkeit der Haarbalgdrüsen ausnützt zur Speicherung von Wirkstoffen und ihrer Freisetzung über das Sebum, wobei die Zusammensetzung mindestens umfasst:
- ein Vektor-Lösungsmittel zur Diffusion der Wirkstoffe der Zusammensetzung an die Hautoberfläche und
- mindestens einen Komplex aus essentiellen Ölen,
b) Diffusion Wirkstoffen der Zusammensetzung und
c) Speicherung der Wirkstoffe der Zusammensetzung durch die Haarbalgdrüsen und deren Freisetzung.

2. Verfahren nach Anspruch 1, **dadurch gekennzeichnet, dass** das Vektor-Lösungsmittel Ethoxydiglykol ist.

3. Verfahren nach Anspruch 1, **dadurch gekennzeichnet, dass** das Vektor-Lösungsmittel ausgewählt ist aus der Gruppe:
Aceton, Acetonitril, Benzylalkohol, Butyldiglykol, Dimethylacetamid, Dimethylformamid, Dipropylenglykol-n-butylether, Ethanol, Isopropanol, Methanol, Ethylenglykolmonoethylether, Monomethylacetamid, Dipropylenglykolmonomethylether, flüssige Polyoxyethylenglykole, Propylenglykol, 2-Pyrrolidon, Diethylenglykolmonoethylether, Ethylenglykol, Diethylphthalat und einer Mischung aus mindestens zwei dieser Verbindungen.

4. Verfahren nach den Ansprüchen 1, 2 und 3, **dadurch gekennzeichnet, dass** die Zusammensetzung:
einen aus einem organischen Lösungsmittel gebildeten Vektor,
einen Komplex aus essentiellen Ölen und
mehrfach ungesättigte Fettsäuren der lokal bei Tieren angewandt werden, umfasst.

5. Verfahren nach Anspruch 4, **dadurch gekennzeichnet, dass** die Fettsäuren aus Hanfölen ausgewählt sind und in einem Anteil von 1 bis 15 % vorhanden sind.

6. Verfahren nach Anspruch 4, **dadurch gekennzeichnet, dass** die Fettsäuren durch Produkte zugeführt werden, die aus der folgenden Gruppe ausgewählt sind:
Fischöl (Kaiserbarsch oder Orange Roughy)
Borretschöl (Borago officinalis)
Kokosöl (Cocos nucifera)
Kukuiöl
Sesamöl (Sesamum indicum)
wiederhergestellte synthetische oder extrahierte natürliche, mehrfach
ungesättigte Fettsäuren (AGPI)
Eicopentaensäure (EPA) und
Docosahexaensäure (DHA),
Karitebutter (Shea Butter)
Olivenöl (Olea europea)
Rapsöl
Nussöl
Sojaöl
Buglosseöl (Echium plantagineum)
in einem Anteil von 3 bis 50 %.

7. Verfahren nach Anspruch 6, worin der Gehalt der genannten Fettsäuren zwischen 5 und 10 % beträgt.

8. Verfahren nach irgendeinem der vorhergehenden Ansprüche, **dadurch gekennzeichnet, dass** die Zusammensetzung einen die Kristallisation inhibierenden, verdickenden und stabilisierenden Filmbildner umfasst.

9. Verfahren nach dem vorhergehenden Anspruch, worin der Filmbildner ein Polyvinylpyrrolidon ist oder aus der Gruppe ausgewählt ist:
Hydroxymethylcellulose, Polyvinylalkohole, Vinylacetat-Vinylpyrrolidon-Copolymere, Polyethylenglykole, Benzylalkohol, Mannitol, Glycerol, Sorbitol, polyoxy-ethylierte Sorbitanester, Lecithin, Natriumcarboxymethylcellulose und Acrylderivate, wie Methacrylate und dergleichen.

10. Verfahren nach irgendeinem der vorhergehenden Ansprüche, **dadurch gekennzeichnet, dass** die Zusammensetzung in Form einer Retard-Formulierung vorliegt, die eine verlängerte Freisetzung der Wirkstoffe ermöglicht.

11. Verfahren nach dem vorhergehenden Anspruch, **dadurch gekennzeichnet, dass** die Retard-Formulierung umfasst:
Siliciumdioxid und/oder
Beta-cyclodextrine, cyclische Zucker natürlichen Ursprungs, die in der Formulierung in einer Konzentration von 2 bis 10 % enthalten sind,
und/oder aus Holz gewonnene Cellulosefasern, die in der Formulierung in einer Konzentration von 2 bis 10 % enthalten sind,
und/oder Kügelchen aus Polymethylmethacrylat und Nylon, die in der Formulierung in einer Konzentration von 4 bis 12 % enthalten sind,
und/oder umhüllte oder nichtumhüllte Siliciumdioxid-Mikroreservoirs, die in der Formulierung in einer Konzentration von 2 bis 10 % enthalten sind,
und/oder okklusive und rückhaltende lineare Polyesterpolymere mit einer Molekülmasse von 800 bis 5000 Dalton, die in der Formulierung in einer Konzentration von 10 bis 20 % enthalten sind.

12. Verfahren nach irgendeinem der vorhergehenden Ansprüche, worin die Zusammensetzung derart bemessen wird, dass, wenn die hautkosmetische Zusammensetzung auf eine oder mehrere Stellen des Fells des Tieres aufgebracht wird, sie eine Behandlung auf der gesamten Hautoberfläche des Tieres während mehrerer Tage bewirkt.

13. Verfahren nach irgendeinem der vorhergehenden Ansprüche, worin das Tier ein Haustier ist.
